# EUROPEAN PATENT APPLICATION

(11) **EP 4 850 687 A1**
(43) Date of publication of application: **09.12.2026**
(21) Application number: 25180294.8
(22) Date of filing: 02.06.2025
(51) Int. Cl.: A61K 39/108, A61P 31/04

(54) **ORAL VACCINE AGAINST MULTIPLE DIARRHEA-CAUSING PATHOGENS**

(71) Applicant: Scandinavian Biopharma Holding AB, 171 48 Solna (SE)
(72) Inventor: SJÖSTRAND, Björn, 168 50 Bromma (SE); CARLIN, Nils, 168 39 Bromma (SE)
(74) Representative: Novitas Patent AB

(57) **Abstract**

A vaccine comprising effective amounts of i) *E. coli* colonization factor antigens comprising CFA/I, CS3, CS5 and CS6 antigens, present on inactivated whole *Escherichia coli* cells; ii) a heat labile enterotoxin B subunit antigen being an *LCTBA* protein; and iii) an adjuvant being double mutant (R192G/ L211A) of *E. coli* heat-labile toxin (dmLT), for use in inducing protective immunity against diarrheal disease caused by *Campylobacter spp.* and/or Enteroaggregative *Escherichia coli* (EAEC).

## Description

### TECHNICAL FIELD

The present invention relates to oral vaccines for use in the prevention of diarrheal disease caused by *Campylobacter spp.* and/or Enteroaggregative *Escherichia coli* (EAEC).

### BACKGROUND TO THE INVENTION

The global diarrheal disease burden remains high, with approximately four billion cases estimated to occur annually in all age groups, with the highest incidence among infants and young children under five years of age. In this age group, enteric infections result in nearly 600,000 deaths each year, comprising approximately 9 percent of global child deaths and extracting an enormous physical and economic toll in low resource countries. Indeed, diarrhea is second only to pneumonia among the leading causes of death due to disease in children under five years old. Diarrhea can also be a triggering event for death from other causes, particularly pneumonia. Despite encouraging declines in overall diarrhea-associated mortality since year 2000, incidence rates remain high and have changed very little, even with incremental improvements in sanitation and water quality.

Travelers' diarrhea (TD) is the most common travel-related illness with per trip attack rate estimates of 10-40%. TD is a clinical syndrome that can result from a variety of intestinal pathogens. The predominant enteropathogens are bacteria that are considered to account for more than 80%-90% of cases. Overall, the most common bacterial pathogen identified is enterotoxigenic *Escherichia coli* (ETEC), followed by *Campylobacter jejuni, Shigella spp.,* and *Salmonella spp.* Enteroaggregative and other *E. coli* pathotypes also are commonly found in cases of TD. ETEC is estimated to cause approximately 35 million episodes of travelers' diarrhea yearly.

Among the primary infectious causes of diarrheal disease, enterotoxigenic *E. coli* (ETEC) is one of the most important pathogens for which there is currently no licensed vaccine. ETEC has been a long-standing World Health Organization (WHO) target for vaccine development. ETEC is also among the most frequent bacterial causes of diarrhea among travelers to Africa, Asia, and Latin America, including military personnel deployed to these areas.

Campylobacter-associated diarrheal disease is estimated to cause more than 160 million cases of gastroenteritis every year and up to 1 out of 4 cases of travelers' diarrhea especially in South and East Asia. Campylobacter is transmitted to humans from animals/animal products. Virtually all human illness is caused by one or two species (*C*. *coli* and *C. jejuni*). The Centers for Disease Control reports that 99% of illness is caused by C. jejuni while other studies have shown that globally, more than 90% of Campylobacter infections are caused by *C. jejuni,* followed by *C*. *coli* with 5-10%. The stunting consequences of a Campylobacter infection can be severe, and the bacteria cause nearly 200 deaths per year in the US. Current data show that 1 of every 1000 people infected with the bacteria develop an acute progressively paralyzing neurological condition known as Guillain Barré Syndrome (GBS). GBS causes every muscle to shut down (including the muscles that are necessary for breathing), often leading to intubation within a few days. The patients' neurological symptoms are slowly reversed, but the rehabilitation phase can vary from months to many years. Depending on the patients' previous symptoms and state, the condition can be deadly. More commonly, 10% to 14% % of all patients develop irritable bowel syndrome (IBS) within 1-2 years after a Campylobacter infection. Sanitation and hygiene are the current standard of care for prevention, and antibiotics remain the only viable therapy, contributing to the increased spread of anti-microbial resistant strains. Preventive measures have been proposed to eliminate the spread of Campylobacter infection and vaccine development is one important component of these preventive measures.

Enteroaggregative *Escherichia coli* (EAEC) is increasingly recognized as a cause of both acute and persistent diarrhea particularly in children in developing countries, travelers visiting less-developed areas, immunocompromised individuals and in industrialized countries during foodborne outbreaks. The exact global number of cases yearly is not well understood due to underreporting and diagnostic limitations. It is estimated that EAEC contribute to millions of diarrheal cases annually, especially in endemic regions. E AEC is associated with persistent diarrhea, which can lead to significant morbidity and mortality, especially in vulnerable populations such as children. Treatment typically involves rehydration and, in severe cases, treatment with antibiotics. However, antibiotic resistance is a growing concern. Current medical needs include improved diagnostics, improved public health measures and novel treatment and vaccines.

### Clinical development of ETVAX

ETVAX is a new multivalent, second-generation vaccine which is now under clinical development (WO2013037397A1). This improved vaccine contains 4 different inactivated *E*. *coli* strains expressing the most prevalent ETEC colonization factors (CFs), i.e. CFA/I, CS3, CS5 and CS6, in a total of approx. 8x10¹⁰ bacteria + 1 mg *LCTBA* and a mucosal adjuvant, i.e. double mutated LT (dmLT) (10 µg/dose) in a "full"/"normal" unit dose. Overall, ETEC vaccine administered with dmLT was safe and well tolerated when studied in several phase I and II clinical studies. No serious AEs were identified, and the recorded AEs were mainly mild. Immune responses analysed showed the tetravalent ETEC vaccine to be strongly immunogenic with intestinal (fecal) antibody responses and the intestine-derived antibody secreting cell (ALS) responses against the five primary antigens (LTB, CFA/I, CS3, CS5 and CS6) significantly higher in both frequency and magnitude in all three vaccine groups compared with the placebo group.

ETVAX has been studied in a randomized, placebo-controlled phase IIb study to evaluate safety, immunogenicity, diagnostic methodology, and to estimate vaccine efficacy for prevention of ETEC diarrhea in 749 healthy adult travelers visiting West Africa.

### Object of the present invention

An object of the present invention is the provision of new methods for the reducing the risk of diarrheal disease caused by *Campylobacter spp.* and/or enteroaggregative *Escherichia coli* (EAEC). Another object is the provision of new uses for known vaccine compositions.

### DEFINITIONS

The term **antigen** refers to structures to which antibodies generated by the adaptive immune system of vertebrates specifically bind, or to which specific T-cell responses are elicited. Antigens are often proteins, peptides or polysaccharides (sometimes containing a lipid moiety). In the context of vaccines, an antigen is an ingredient in a vaccine against which a specific antibody and/or T-cell response is intended to be elicited in the host to which the vaccine is administered. Some vaccines contain several antigens. In the vaccine context, a vaccine antigen generally bears a structural resemblance to a pathogen antigen present in a pathogen against which the vaccine is intended to be used. A vaccine antigen may be a fragment or a modified version of the pathogen antigen, or a complete pathogen antigen presented in a non-pathogenic form e.g. as an inactivated/weakened pathogen or a recombinant protein produced in a non-pathogenic host.

The term **adjuvant** refers to a component added to a vaccine or other immunogenic composition that increases the effectiveness of the immune response to the antigen present in the vaccine or the other immunogenic composition. To be clear, the role as an adjuvant does not exclude an additional role as an antigen.

The term **protective immunity** in the present context refers to immunization measures resulting in any degree of reduction in the likelihood of developing the condition for which the protective immunity is relevant, including a minor, substantial or major reduction in likelihood of developing the condition as well as total prevention. Preferably, the degree of likelihood reduction is at least a minor reduction.

**Colonization factors (CFs)** are major virulence factors of enterotoxigenic *Escherichia coli* (ETEC). In addition to colonization factors, ETEC virulence factors include heat-stable (ST) and heat-labile (LT) toxins. The CFs are either fimbrial, fibrial, or nonfimbrial in structure, and facilitate the adhesion of the bacteria to the intestinal cells and enable the bacteria to colonize the host. After colonization of the small intestine the bacteria secrete heat-labile toxin (LT), heat stable-toxin (ST) or both LT and ST which cause diarrhea. The morphology of the colonization factors is often determined by electron microscopy or predictions based on protein sequence.

The **CFA/I antigen, CS3 antigen, CS5 antigen** and **CS6 antigen** are prominent colonization factors present in ETEC isolates. A degree of biological sequence diversity exists in different strains and isolates and the sequence of an antigen useful in vaccines may differ somewhat from the reference sequence, as long as the antigen is capable of eliciting a clinically relevant immune response to the relevant colonization factor. The antigens have preferably at least 80%, or in increasing order of preference at least 85%, at least 95%, at least 98%, or complete sequence identity to their respective reference sequences cited below. In the context of designing ETEC vaccines, the antigens elicit the following relevant immune reactivity :
- A CFA/I antigen is capable of eliciting immune reactivity against CFA/I expressing ETEC strains.
- A CS3 antigen is capable of eliciting immune reactivity against CS3 expressing strains such as CS1 + CS3, CS2+ CS3 and CS3 only expressing ETEC strains.
- A CS5 antigen is capable of eliciting immune reactivity against CS5 expressing strains such as CS5 + CS6 expressing ETEC strains.
- A CS6 antigen is capable of eliciting immune reactivity against CS6 expressing strains such as CS4 + CS6, CS5 + CS6 and CS6 only expressing ETEC strains.

The **CFA/I (Colonization Factor Antigen I)** is a fimbrial structure, which allows it to attach to the intestinal lining. The fimbriae are composed of two main subunits. The CfaB (major repeating subunit) forms the bulk of the fimbriae and provides the repeating structural unit and the CfaE (single tip subunit) located at the tip of the fimbriae plays a role in initial attachment to host cells. See Jordi BJ, Willshaw GA, van der Zeijst BA, Gaastra W. The complete nucleotide sequence of region 1 of the CFA/I fimbrial operon of human enterotoxigenic Escherichia coli. DNA Seq. 1992;2(4):257-63 for an introduction to CFA/1. Reference sequences of cfaB and cfaE are published in Genbank accession number M55661.

The **CS3** (Coli Surface Antigen 3) is part of the fibrial family of colonization factors comprising of two subunits cstH and cstG forming the CS3 fimbriae. See Jalajakumari MB, Thomas CJ, Halter R, Manning PA. Genes for biosynthesis and assembly of CS3 pili of CFA/II enterotoxigenic Escherichia coli: novel regulation of pilus production by bypassing an amber codon. Mol Microbiol. 1989;3(12):1685-95 for an introduction to CS3. A reference sequence of cstH and cstG are described in US11,077,200 B2 (cstG - SEQ ID NO: 87. cstH - SEQ ID NO: 85).

The **CS5** (Coli Surface Antigen 5) is a helical with two fimbria (csfA and csfD) arranged into a helix colonization factor expressed by certain ETEC strains. CS5 is one of the most common CFs worldwide, along with CS6. See Duthy TG, Staendner LH, Manning PA, Heuzenroeder MW. CS5 Pilus Biosynthesis Genes from Enterotoxigenic Escherichia coli O115:H40. J Bacteriol [Internet]. 1999;181(18):5847-51 for an introduction to CS5. Reference sequences of csfA and csfD are published in Genbank accession number AJ224079.

The **CS6** (Coli Surface Antigen 6) is a nonfimbrial outer membrane protein colonization factor. CS6 is a polymer of two protein subunits CssA and CssB, CssB binds to sulfatide and recognizes the extracellular matrix protein fibronectin, and CssA shows general adhesiveness. See Wolf MK, de Haan LA, Cassels FJ, Willshaw GA, Warren R, Boedeker EC, et al. The CS6 colonization factor of human enterotoxigenic Escherichia coli contains two heterologous major subunits. FEMS Microbiol Lett. 1997;148(1):35-42 for an introduction to CS6. Reference sequences of cssA and cssB are published in Genbank accession number U04844.

The synonymous terms ***LCTBA*** and ***LCTBA*-protein** refer to a hybrid protein between the B-subunit of the *E. coli* heat-labile enterotoxin (LTB) and the B-subunit of the cholera toxin (CTB). Seven amino acids in the CTB molecule have been replaced by amino acids at corresponding positions of the LTB molecule. For details on LCTBA, see Lebens M, Shahabi V, Backstrom M, et al. 1996. Synthesis of hybrid molecules between heat-labile enterotoxin and cholera toxin B subunits: potential for use in a broad-spectrum vaccine. Infect Immun 64:2144-2150) and WO1996034893A1.

**dmLT** is a derivative of enterotoxigenic *Escherichia coli* heat labile toxin (LT) that has been genetically modified by replacing the arginine at amino acid position 192 with glycine and the leucine at amino acid position 211 with alanine. For an introduction to dmLT see, John D. Clements and Elizabeth B. Norton The Mucosal Vaccine Adjuvant LT(R192G/L211A) or dmLT mSphere. 2018; Jul-Aug; 3(4): e00215-18*,* WO1996006627A1, WO1999047167A1 and US 6,033, 673). The protein has been synonymously designated LT(R192G/L211A) in the literature and has been extensively evaluated in pre-clinical animal studies for its ability to adjuvant the immune responses for co-administered antigens. For characterization see Norton et al. (Norton EB, Lawson LB, Freytag LC, Clements J D. Characterization of a mutant Escherichia coli heat-labile toxin, LT(R192G/L211A), as a safe and effective oral adjuvant. Clin Vaccine Immunol. 2011 Apr; 18(4):546-51.).

The term ***sequence identity*** expressed in percentage is defined as the value determined by comparing two optimally aligned sequences over a comparison window, wherein a portion of the sequence in the comparison window may comprise additions or deletions (i.e., gaps) as compared to the reference sequence (which does not comprise additions or deletions) for optimal alignment of the two sequences. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Unless indicated otherwise, the comparison window is the entire length of the sequence being referred to. In this context, optimal alignment is the alignment produced by the BLASTP algorithm as implemented online by the US National Center for Biotechnology Information (see The NCBI Handbook, 2nd edition [https://www.ncbi.nlm.nih.gov/books/NBK143764/ ]), with the following input parameters: Word length=3, Matrix=BLOSUM62, Gap cost=11, Gap extension cost=1.

***Campylobacter spp*.** is a genus of Gram-negative, motile, spiral-shaped, microaerophilic bacteria recognized as a leading cause of bacterial gastroenteritis in humans worldwide, and also infects a wide range of animals, posing a significant zoonotic risk. Over campylobacter 20 species have been identified, with the following being the most clinically significant:
- *Campylobacter jejuni,* responsible for about 90% of human *Campylobacter* infections. This species commonly colonizes poultry asymptomatically, leading to meat contamination during slaughter.
- *Campylobacter coli,* accounts for approximately 5-10% of human cases. This species is frequently associated with contaminated pork and poultry.
- *Campylobacter fetus* is a notable cause of abortions in sheep and cattle.

*A Campylobacter* infection causes campylobacteriosis, in humans characterized by diarrhea, abdominal cramps, fever, nausea and vomiting, headache, muscle pain and dehydration. **Enteroaggregative Escherichia coli (EAEC)** is a distinct pathotype of *E. coli* with several unique features in its "stacked-brick" adherence pattern, biofilm formation, expression of fimbrial structures that that are not typically found in other *E. coli* pathotypes, and autoagglutination in liquid culture, meaning they clump together without the need for host cells. As a proxy or complement to the defining phenotypical characteristics, genetic markers may be used in identifying EAEC. These include *aggR* (transcriptional regulator), *aafA* (AAF structural subunit), *cap* (dispersin), *pet* (plasmid-encoded toxin), and *set1A* (Shigella enterotoxin 1), which are typically encoded on the aggregative adherence (pAA) plasmid, and *astA* (EAST1 toxin), which is often plasmid-encoded but may occasionally be chromosomal. However, it should be understood that EAEC is genetically diverse, and atypical strains might lack one or more of the genetic markers while falling withing the phenotypical definition. EAEC is by definition distinct from other *E. coli* pathotypes such as enteropathogenic *E. coli* (EPEC), enterotoxigenic *E. coli* (ETEC), enterohemorrhagic *E. coli* (EHEC), and enteroinvasive *E. coli* (EIEC). EAEC is commonly associated with persistent diarrhea (lasting more than 14 days), especially in children in developing countries, immunocompromised and in travelers as a cause of traveler's diarrhea.

### SUMMARY OF THE INVENTION

The inventors have unexpectedly discovered that the vaccine candidate being developed for the prevention of diarrheal disease caused by enterotoxigenic *Escherichia coli* (ETEC) also resulted in significant protective effect against diarrheal disease caused by *Campylobacter spp.* and Enteroaggregative *Escherichia coli* (EAEC).

The present invention relates to the following items. The subject matter disclosed in the items below should be regarded disclosed in the same manner as if the subject matter were disclosed in patent claims.
1. An oral vaccine comprising effective amounts of:
   a. *E. coli* colonization factor antigens comprising CFA/I, CS3, CS5 and CS6 antigens, present on inactivated whole *Escherichia coli* cells;
   b. a heat labile enterotoxin B subunit antigen being an *LCTBA* protein; and
   c. an adjuvant being double mutant (R192G/ L211A) of *E. coli* heat-labile toxin (dmLT);
   for use in inducing protective immunity against diarrheal disease caused by *Campylobacter spp.* and/or enteroaggregative *Escherichia coli* (EAEC).
2. The vaccine for use according to item 1, wherein the disease is caused by *Campylobacter spp.*
3. The vaccine for use according to item 2, wherein the disease is caused by *Campylobacter jejuni.*
4. The vaccine for use according to item 2 or 3, wherein the disease is caused by *Campylobacter coli.*
5. The vaccine for use according to item 1, wherein the disease is caused by EAEC.
6. The vaccine for use according to any of the preceding items, wherein the vaccine is further for use in inducing protective immunity against diarrheal disease caused by enterotoxigenic *E. coli* (ETEC).
7. The vaccine for use according to any of the preceding items, wherein the vaccine comprises inactivated whole *Escherichia coli* cells in an amount of at least about 1.0 x 10¹⁰ cells per unit dose, preferably at least about 2.0 x 10¹⁰ cells per unit dose, more preferably at least about 4 x 10¹⁰ cells per unit dose, most preferably at least about 4.8 x 10¹⁰ cells per unit dose.
8. The vaccine for use according to any of the preceding items, wherein the vaccine comprises inactivated whole *Escherichia coli* cells in an amount of less than 12 x 10¹⁰ cells per unit dose, preferably less than 11 x 10¹⁰ cells per unit dose, most preferably no more than 10 x 10¹⁰ cells per unit dose.
9. The vaccine for use according to any of the preceding items, wherein the vaccine comprises dmLT in an amount of at least about 1.0 µg per unit dose, preferably at least about 2 µg per unit dose, more preferably at least about 4 µg per unit dose, most preferably at least about 8 µg per unit dose.
10. The vaccine for use according to any of the preceding items, wherein the vaccine comprises *LCTBA* protein in an amount of at least about 0.1 mg per unit dose, preferably at least about 0.2 mg per unit dose, more preferably at least about 0.4 mg per unit dose, most preferably at least about 0.8 mg per unit dose.
11. The vaccine for use according to any of the preceding items, wherein the vaccine comprises CFA/I in an amount of at least about 0.04 mg per unit dose, preferably at least about 0.08 mg per unit dose, more preferably at least about 0.15 mg per unit dose, most preferably at least about 0.3 mg per unit dose.
12. The vaccine for use according to any of the preceding items, wherein the vaccine comprises CS3 in an amount of at least about 0.13 mg per unit dose, preferably at least about 0.25 mg per unit dose, more preferably at least about 0.5 mg per unit dose, most preferably at least about 1.0 mg per unit dose.
13. The vaccine for use according to any of the preceding items, wherein the vaccine comprises CS5 in an amount of at least about 0.03 mg per unit dose, preferably at least about 0.05 mg per unit dose, more preferably at least about 0.1 mg per unit dose, most preferably at least about 0.2 mg per unit dose.
14. The vaccine for use according to any of the preceding items, wherein the vaccine comprises CS6 in an amount of at least about 0.006 mg per unit dose, preferably at least about 0.013 mg per unit dose, more preferably at least about 0.025 mg per unit dose, most preferably at least about 0.05 mg per unit dose.
15. The vaccine for use according to any of the preceding items, wherein the vaccine comprises, per unit dose:
   a. inactivated whole *Escherichia coli* cells comprising the following antigens:
      i. 0.3-1.6 mg CFA/I;
      ii. 1.0-5.2 mg CS3;
      iii. 0.2-1.1 mg CS5; and
      iv. 0.05-0.24 mg CS6;
   b. 0.8-1.2 mg of LCBTA protein; and
   c. 8-14 µg of dmLT adjuvant.
16. The vaccine for use according to item 14, wherein the unit dose comprises 4.0 x 10¹⁰ to 12 x 10¹⁰ whole *Escherichia coli* cells.
17. The vaccine for use according to item 14 or 15, for use in immunizing human subjects of at least 6 years of age.
18. The vaccine for use according to any of items 1-13, wherein the vaccine comprises, per unit dose:
   a. inactivated whole *Escherichia coli* cells comprising the following antigens:
      i. 0.15-0.8 mg CFA/I;
      ii. 0.5-2.6 mg CS3;
      iii. 0.1-0.55 mg CS5; and
      iv. 0.025-0.12 mg CS6;
   b. 0.4-0.6 mg of LCBTA protein; and
   c. 4-7 µg of dmLT adjuvant.
19. The vaccine for use according to item 17, wherein the unit dose comprises 2.0x 10¹⁰ to 6.0 x 10¹⁰ whole *Escherichia coli* cells.
20. The vaccine for use according to item 17 or 18, for use in immunizing human subjects of 2-5 years of age.
21. The vaccine for use according to any of items 1-13, wherein the vaccine comprises, per unit dose:
   a. inactivated whole *Escherichia coli* cells comprising the following antigens:
      i. 0.075-0.4 mg CFA/I;
      ii. 0.25-1.3 mg CS3;
      iii. 0.05-0.275 mg CS5; and
      iv. 0.0125-0.06 mg CS6;
   b. 0.2-0.3 mg of LCBTA protein; and
   c. 2-3.5 µg of dmLT adjuvant.
22. The vaccine for use according to item 20, wherein the unit dose comprises 1.0x 10¹⁰ to 3.0 x 10¹⁰ whole cells.
23. The vaccine for use according to item 20 or 21, for use in immunizing human subjects of 6-23 months of age.
24. The vaccine for use according to any of the preceding items, wherein the vaccine comprises inactivated *E. coli* cells comprising CS6 inactivated by phenol.
25. The vaccine for use according to any of the preceding items, wherein the vaccine is for administration in at least two doses separated in time by 7-30 days, preferably 14±7 days to a subject.
26. The vaccine for use according to any of items 1-24, wherein the vaccine is for administration in at least three doses to a subject, wherein the first two doses are separated in time by 7-42 days, and wherein the third dose is administered 50-130 days after the first dose. The vaccine for use according to any of the preceding items, wherein the vaccine is for use in oral administration to a subject in a buffer suitable for neutralizing gastric acid.
27. The vaccine for use according to any of the preceding items, wherein the vaccine is for use in a human subject.
28. The vaccine for use according to any of the preceding items, wherein the vaccine is for use in an adult human of at least 16 years of age.
29. The vaccine for use according to any of the preceding items, wherein the vaccine is for use in a human of 6-15 years of age.
30. The vaccine for use according to any of the preceding items, wherein the vaccine is for use in a human of 2-5 years of age at half of the unit dose.
31. The vaccine for use according to any of the preceding items, wherein the vaccine is for use in a human of 6-23 months of age at quarter of the unit dose.

### DETAILED DESCRIPTION

The present invention provides an oral vaccine comprising effective amounts of:
a. *E. coli* colonization factor antigens comprising CFA/I, CS3, CS5 and CS6 antigens, present on inactivated whole *Escherichia coli* cells;
b. a heat labile enterotoxin B subunit antigen being an *LCTBA* protein; and
c. an adjuvant being double mutant (R192G/ L211A) of *E. coli* heat-labile toxin (dmLT);
for use in inducing protective immunity against diarrheal disease caused by *Campylobacter spp.* and/or enteroaggregative *Escherichia coli* (EAEC).

The diarrheal disease may be caused *by Campylobacter spp.* The disease may be caused by *Campylobacter jejuni* and/or *Campylobacter coli.* Alternatively, the diarrheal disease may be caused by EAEC. The vaccine may further be for use in inducing protective immunity against diarrheal disease caused by enterotoxigenic *E. coli* (ETEC).

The *E. coli* colonization factor antigens may be present on inactivated whole bacterial cells, preferably *E. coli* cells. The various antigens may be presented on different cells. Alternatively, a cell type may present several of the antigens.

The vaccine may comprise inactivated *E. coli* cells comprising CS6, inactivated by phenol. Suitable inactivation methods are disclosed in WO2013037718 A1. Other possible inactivation methods include formalin inactivation.

### Vaccine unit dose

The vaccine for the claimed use is normally provided in a unit dose. The amount of vaccine components in a unit dose may vary depending on the subject to be immunized. A unit dose intended for use in immunizing an adult human being at least 16 years of age as well as children and adolescents aged 6-15 years of age is considered a "full" unit dose or a "normal" unit dose. A unit dose for use in immunizing a human child of 2-5 years of age may contain half the amount of components compared to a full dose. A unit dose for use in immunizing a human infant of 6-23 months of age may contain a quarter of the amount of components compared to a full dose.

The vaccine may comprise inactivated whole *Escherichia coli* cells in an amount of at least about 1.0 x 10¹⁰ cells per full unit dose, preferably at least about 2.0 x 10¹⁰ cells per full unit dose, more preferably at least about 4 x 10¹⁰ cells per full unit dose, most preferably at least about 4.8 x 10¹⁰ cells per full unit dose. The vaccine may comprise inactivated whole *Escherichia coli* cells, in an increasing order of preference, the following number of cells per full unit dose: about 0.8-14 x 10¹⁰, about 4.0-12 x 10¹⁰, about 4.8-10.0 x 10¹⁰, about 7.2-8.8 x 10¹⁰, about 8.0 x 10¹⁰. The vaccine may comprise inactivated whole *Escherichia coli* cells in an amount of less than 12 x 10¹⁰ cells per full unit dose, preferably less than 11 x 10¹⁰ cells per full unit dose, most preferably no more than 10 x 10¹⁰ cells per full unit dose. For unit dose for use in human child of 2-5 years of age, half the aforesaid amounts of components are applicable, *mutatis mutandis.* For a unit dose for use in a human infant of 6-23 months of age, a quarter of the aforesaid amounts are applicable, *mutatis mutandis.*

The vaccine may comprise dmLT in an amount of at least about 1.0 µg per full unit dose, preferably at least about 2 µg per full unit dose, more preferably at least about 4 µg per full unit dose, most preferably at least about 8 µg per full unit dose. The vaccine may comprise, in an increasing order of preference, the following amount of dmLT per full unit dose: about 2.0-17.5 µg, about 4-15 µg, about 8-14 µg, about 9-11 µg, about 10 µg. For a unit dose for use in human child of 2-5 years of age, half the aforesaid amounts of components are applicable, *mutatis mutandis.* For a unit dose for use in a human infant of 6-23 months of age, a quarter of the aforesaid amounts are applicable, *mutatis mutandis.*

The vaccine may comprise *LCTBA* protein in an amount of at least about 0.1 mg per full unit dose, preferably at least about 0.2 mg per full unit dose, more preferably at least about 0.4 mg per full unit dose, most preferably at least about 0.8 mg per full unit dose. The vaccine may comprise, in an increasing order of preference, the following amount of *LCTBA* protein per full unit dose: about 0.20-1.5 mg, about 0.4-1.5 mg, about 0.8-1.2 mg, about 0.9-1.1 mg, about 1.0 mg. For a unit dose for use in human child of 2-5 years of age, half the aforesaid amounts of components are applicable, *mutatis mutandis.* For a unit dose for use in a human infant of 6-23 months of age, a quarter of the aforesaid amounts are applicable, *mutatis mutandis.*

The vaccine may comprise colonization factor CFA/I in an amount of at least about 0.04 mg per full unit dose, preferably at least about 0.08 mg per full unit dose, more preferably at least about 0.15 mg per full unit dose, most preferably at least about 0.3 mg per full unit dose. The vaccine may comprise, in an increasing order of preference, the following amount of CFA/I per full unit dose: about 0.075-1.7 mg, about 0.15-1.6 mg, about 0.3-1.6 mg, about 0.9-1.1 mg, about 0.98 mg. For a unit dose for use in human child of 2-5 years of age, half the aforesaid amounts of components are applicable, *mutatis mutandis.* For a unit dose for use in a human infant of 6-23 months of age, a quarter of the aforesaid amounts are applicable, *mutatis mutandis.*

The vaccine may comprise colonization factor CS3 in an amount of at least about 0.13 mg per full unit dose, preferably at least about 0.25 mg per full unit dose, more preferably at least about 0.5 mg per full unit dose, most preferably at least about 1.0 mg per full unit dose. The vaccine may comprise, an increasing order of preference, the following amount of CS3 per full unit dose: about 0.25-7.5 mg, about 0.5-6.0 mg, about 1.0-5.2 mg, about 2.8-3.4 mg, about 3.13 mg. For a unit dose for use in human child of 2-5 years of age, half the aforesaid amounts of components are applicable, *mutatis mutandis.* For a unit dose for use in a human infant of 6-23 months of age, a quarter of the aforesaid amounts are applicable, *mutatis mutandis.*

The vaccine may comprise colonization factor CS5 in an amount of at least about 0.03 mg per full unit dose, preferably at least about 0.05 mg per full unit dose, more preferably at least about 0.1 mg per full unit dose, most preferably at least about 0.2 mg per full unit dose. The vaccine may comprise, in an increasing order of preference, the following amount of CS5 per full unit dose: about 0.05-2.0 mg, about 0.1-1.5 mg, about 0.2-1.1 mg, about 0.6-0.7 mg, about 0.63 mg. For a unit dose for use in human child of 2-5 years of age, half the aforesaid amounts of components are applicable, *mutatis mutandis.* For a unit dose for use in a human infant of 6-23 months of age, a quarter of the aforesaid amounts are applicable, *mutatis mutandis.*

The vaccine may comprise colonization factor CS6 in an amount of at least about 0.006 mg per full unit dose, preferably at least about 0.013 mg per full unit dose, more preferably at least about 0.025 mg per full unit dose, most preferably at least about 0.05 mg per full unit dose. The vaccine may comprise, in an increasing order of preference, the following amount of CS6 per full unit dose: about 0.0125-0.5 mg, 0.025-0.5 mg, 0.05-0.24 mg, 0.13-0.16 mg, about 0.145 mg. For a unit dose for use in human child of 2-5 years of age, half the aforesaid amounts of components are applicable, *mutatis mutandis.* For a unit dose for use in a human infant of 6-23 months of age, a quarter of the aforesaid amounts are applicable, *mutatis mutandis.*

In an embodiment, the vaccine comprises, per unit dose:
a. inactivated whole *Escherichia coli* cells comprising the following antigens:
   i. 0.3-1.6 mg CFA/I;
   ii. 1.0-5.2 mg CS3;
   iii. 0.2-1.1 mg CS5; and
   iv. 0.05-0.24 mg CS6;
b. 0.8-1.2 mg of LCBTA protein; and
c. 8-14 µg of dmLT adjuvant.

In this embodiment, the unit dose may comprise 4.0 x 10¹⁰ to 12 x 10¹⁰ whole *Escherichia coli* cells.

The vaccine according to this embodiment may be for use in immunizing adult human subjects being at least 16 years of age as well as children and adolescents being 6-15 years of age.

In another embodiment, the vaccine comprises, per unit dose:
a. inactivated whole *Escherichia coli* cells comprising the following antigens:
   i. 0.15-0.8 mg CFA/I;
   ii. 0.5-2.6 mg CS3;
   iii. 0.1-0.55 mg CS5; and
   iv. 0.025-0.12 mg CS6;
b. 0.4-0.6 mg of LCBTA protein; and
c. 4-7 µg of dmLT adjuvant.

In this embodiment, the unit dose may comprise 2.0x 10¹⁰ to 6.0 x 10¹⁰ whole *Escherichia coli* cells.

The vaccine according to this embodiment may be for use in immunizing human subjects of 2-5 years of age.

In yet another embodiment, the vaccine comprises, per unit dose:
a. inactivated whole *Escherichia coli* cells comprising the following antigens:
   i. 0.075-0.4 mg CFA/I;
   ii. 0.25-1.3 mg CS3;
   iii. 0.05-0.275 mg CS5; and
   iv. 0.0125-0.06 mg CS6;
b. 0.2-0.3 mg of LCBTA protein; and
c. 2-3.5 µg of dmLT adjuvant.

In this embodiment, the unit dose may comprise 1.0x 10¹⁰ to 3.0 x 10¹⁰ whole *Escherichia coli* cells.

The vaccine according to this embodiment may be for use in immunizing human subjects of 6-23 months of age.

### Administration regimen and subjects

The vaccine may be formulated for oral administration to a subject, which is preferably a human.

The vaccine may be for oral administration to a subject in a buffer suitable for neutralizing gastric acid, such as a bicarbonate buffer solution, which may be effervescent.

For vaccines intended for adult human subjects being at least 16 years of age as well as children and adolescents being 6-15 years of age a "full" unit dose or a "normal" unit dose may be administered.

For vaccines intended for children, the unit dose as disclosed above may be reduced to half. For vaccines intended for infants, the unit dose may be reduced to ¼. For avoidance of any ambiguity, this entails that the amounts recited above for unit dose are adjusted accordingly, to arrive to a unit dose for children or unit dose for infants.

The vaccine may be for administration to an adult human being at least 16 years of age. For such subjects, a full unit dose may be administered per occasion. The vaccine may be for administration in at least two doses taken orally about 2-weeks interval (at least 1 week but not more than 6 weeks apart). The immunization should ideally be completed at least 1 week prior to potential exposure to pathogens, if circumstances allow. This is normally possible for travelers but may not always be practicable for subjects living in risk areas.

For children and adolescents aged 6-15, the primary vaccination series may involve two doses taken orally about 2-weeks interval (at least 1 week but not more than 6 weeks apart). The immunization should ideally be completed at least 1 week prior to potential exposure to pathogens.

For children aged 2 to 5 years, the primary vaccination series may involve three doses of half the amount of the adults. The 3 doses are to be administered orally with about 2-weeks interval (at least 1 week but not more than 6 weeks apart) and ideally at least 1 week prior to potential exposure to pathogens.

Human infants being 6 to 23 months, the primary vaccination series may involve three doses of 1/4 the amount of the full (adult) unit dose.

For human infants, the vaccine is preferably for use in a method comprising administration in at least three doses to the subject, wherein the day of the first dose is denoted day 1, the second dose is administered on day 8-42 following the first dose and the third dose is administered on day 50-130 following the first dose, with the proviso that the third dose is administered at least 30 days after the second dose. The immunization should ideally be completed at least 1 week prior to potential exposure to pathogens.

### General statements relating to the present disclosure

The term "comprising" is to be interpreted as including but not being limited to. All references are hereby incorporated by reference. The arrangement of the present disclosure into sections with headings and subheadings is merely to improve legibility and is not to be interpreted limiting in any way, in particular, the division does not in any way preclude or limit combining features under different headings and subheadings with each other.

As used herein, the term "about" refers to a value or parameter herein that includes (and describes) embodiments that are directed to that value or parameter per se. For example, description referring to "about 20" includes description of "20." Numeric ranges are inclusive of the numbers defining the range. Generally speaking, the term "about" refers to the indicated value of the variable and to all values of the variable that are within the experimental error of the indicated value (e.g., within the 95% confidence interval for the mean) or within 10 percent of the indicated value, whichever is greater.

### EXAMPLES

The following examples are not to be regarded as limiting. For further information on the experimental details, the skilled reader is directed to a separate section titled Materials and Methods.

### Example 1: Design on clinical study

A randomised, double-blind, placebo-controlled phase 2b study to evaluate safety, efficacy, and immunogenicity of the adjuvanted ETVAX vaccine in healthy Gambian children has been performed. The study included 4,936 children aged 6 to 18 months at the time of enrolment who were randomised in a 1:1 ratio to receive three doses of either ETVAX with 2.5 µg dmLT or placebo (sodium bicarbonate buffer). In the study, subjects were administered one quarter of an adult dose on study days 1, 15-29, and 90-111. The ETVAX vaccine was given together with 10 mL sodium bicarbonate buffer. Children were monitored up to 24 months. The surveillance occurred at 10 trial clinics among children brought to the clinic for any medical illness.

The primary objective was to evaluate the safety and protective efficacy (PE) of ETVAX against moderate-to-severe acute diarrhoea with ≥4 loose or liquid stools associated with culture-detected ETEC expressing vaccine antigens without presence of Shigella, rotavirus, norovirus GII, and *Cryptosporidium.*

The primary safety endpoint in the study was the occurrence of severe adverse effects (SAEs) among all enrolled children from receipt of the first dose to the end of study. A reactogenicity cohort (first 350 children) underwent active home surveillance for safety events for seven days after each dose.

The primary efficacy endpoint was defined as the first episode of clinically significant (moderate-to-severe) acute diarrhoea with four or more loose or liquid stools associated with culture-detected ETEC expressing LT- or LT/ST-enterotoxin, or ST with at least one CF (CFA/I, CS3, CS5, CS6) from 7 days after receiving the third dose.

The secondary objectives included:
- Further evaluation of PE by diarrhoeal aetiology, presence of co-pathogens / coinfections, and acute diarrhoeal episode with ≥3 loose stools in 24 hours (efficacy)

Moreover, the impact on PE of acute diarrhoeal episodes in the presence or absence of the most frequently detected co-pathogens / coinfections and groups of co-pathogens (bacteria, virus and parasites) was explored.

### In the study the following definitions were used for reporting the severity grade of the Diarrhea:

Diarrhea was defined as ≥4 loose/liquid stools in 24 hours (amended to include ≥3 loose/liquid stools to harmonize with global standards), occurring within 10 days after ≥3 consecutive diarrhea-free days distinguishing acute from prolonged or chronic cases.

Diarrhea severity followed WHO dehydration criteria.
- Moderate diarrhea included vomiting (1-2 times/24 hours), thirst, skin turgor ≤2 seconds, or restlessness/irritability.
- Severe diarrhea included intravenous fluid use, hospitalization, inability to drink, vomiting ≥3 times/24 hours, sunken eyes, lethargy, or skin turgor >2 seconds.

ETEC diarrhoea is defined as at least one of 10 *E. coli*-like colonies obtained from the stool that expressed an enterotoxin (LT- or LT/ST-enterotoxin, or ST with at least one CF [CFA/I, CS3, CS5, CS6]).

### Example 2: Vaccine composition for clinical study

In the study, a tetravalent ETEC vaccine (ETVAX) with *LCTBA* supplemented with an adjuvant, double mutant heat labile toxin (dmLT), and an effervescent powder for oral solution (bicarbonate buffer) was evaluated. The ETEC vaccine (ETVAX) has been described previously (WO2013037397A1, WO2013037718A1) and contains formaldehyde or phenol inactivated recombinant *E. coli* strains (ETEX 21-24) overexpressing colonization factors CFA/I, CS3, CS5, and CS6 and a recombinant protein *LCTBA,* a chimera between *E. coli* heat-labile enterotoxin B subunit (LTB) and Vibrio cholerae cholera toxin B-subunit (CTB). An adjuvant, an inactivated double mutant LT(R192G/ L211A) of wild-type *E. coli* heat-labile toxin (dmLT) is administered with the vaccine. ETVAX is intended to protect against disease by inducing anti-colonization factor immunity that prevents adherence of ETEC bacteria to gut mucosa and anti-LT immunity that prevents binding of the heat-labile enterotoxin to the intestinal epithelium. The immune response elicited by the vaccine has been shown to be enhanced by the use of the dmLT adjuvant.

In the study, the subjects were administered 1/4 of an adult dose of ETVAX vaccine or placebo (Sodium Bicarbonate Buffer) and received three oral vaccine doses on study days 1, 15-29, and 90-111. The vaccine was given together with sodium bicarbonate buffer, which were dissolved in water (75 ml) and 10 ml thereof was mixed with the vaccine suspension prior to oral administration. The buffer was used to prevent degradation of the LCTBA hybrid protein, dmLT and CS3 by the gastric acid.

The same sodium bicarbonate buffer as used to prevent degradation by the gastric acid in the active treatment arm, was used as placebo in the study. The buffer was prepared in the same way as for active.

One full adult ETVAX dose contained up to approx. 8 x 10¹⁰ inactivated bacteria (*E. coli* ETEX21, ETEX22, ETEX23 and ETEX 24) and 1 mg of recombinant protein *LCTBA* and 10 µg of adjuvant dmLT. The detailed composition is described in Table 1.

**Table 1 Composition of ETVAX vaccine - a full adult ETVAX vaccine dose and 1/4 dose given in this study**

| **Ingredients** | **Content in adult dose** | **Content in ¼ dose** |
|---|---|---|
| *E. coli* ETEX 21 | 1 mg CFA/I | 0.25 mg CFA/I |
| *E. coli* ETEX 22 | 3 mg CS3 | 0.76 mg CS3 |
| *E. coli* ETEX 23 | 0.6 mg CS5 | 0.15 mg CS5 |
| *E. coli* ETEX 24 | 0.15 mg CS6 | 0.04 mg CS6 |
| LCTBA | 1 mg | 0.25 mg |
| dmLT | 10 µg | 2.5 µg |
| Phosphate buffered saline | q.s. ad 3.30 ml | q.s. ad 0.83 ml |

To maintain blinding of the study, the investigational products (ETVAX, and placebo) were prepared by the un-blinded trained nurses on the day of vaccination (Day 1, Day 15-29 and Day 90-111) at the vaccination clinic according to written procedures. Participants were required to fast for at least 60 minutes prior to the scheduled time of dosing. Participants fasted for an additional 30 minutes after ingestion of the investigational product or placebo. Each ETEC vaccine and dmLT vials were visually inspected to ensure there are no cracks in the vial and the screw top is fastened tightly. The vial was NOT used if it failed visual inspection. The required volume of ETEC vaccine, supplied as a liquid, was mixed with the required volume of sodium bicarbonate buffer solution on the day of preparation for use on dosing day. The vaccine dose in sodium bicarbonate buffer was stored at room temperature for up to 90 minutes prior to dosing. After addition of dmLT, the vaccine was used within 30 minutes. All drug accountability procedures, including cold chain monitor were documented and were the responsibility of the unblinded study staff.

### Example 3: Vaccine efficacy against Campylobacter and EAEC

When *Campylobacter spp.* was used as an endpoint, regardless of ETEC infections or other co-infections, the incidence among vaccine and placebo recipients of moderate to severe *Campylobacter spp.* cases were 2.9% (63/2184) and 4.3% (93/2177), respectively.
- The vaccine protection afforded was 32.5 % with 95% CI (7.5 to 50.7)

When *Campylobacter coli* was used as an endpoint, regardless of ETEC infections or other co-infections, the incidence among vaccine and placebo recipients of moderate to severe *Campylobacter coli* cases were 1.56% (34/2184) and 3.0% (65/2177), respectively.
- The vaccine protection afforded was 47.9 % with 95% CI (21.4 to 65.4)

When *Campylobacter jejuni* was used as an endpoint, regardless of ETEC infections or other co-infections, the incidence among vaccine and placebo recipients of moderate to severe *Campylobacter jejuni* cases were 1.7% (38/2184) and 2.5% (54/2177), respectively.
- The vaccine protection afforded was 29.9% with 95% CI (-5.8 to 53.5)

When EAEC was used as an endpoint, regardless of ETEC infections or other co-infections, the incidence among vaccine and placebo recipients of moderate to severe EAEC cases was 1.7% (37/2184) and 2.8% (62/2177), respectively.
- The protection afforded was 40.5% with 95% CI (11.0 to 60.2)

In conclusion, the ETVAX vaccine unexpectedly demonstrated prevention of *Campylobacter spp.* and EAEC positive diarrhea with or without co-infections.

### MATERIALS AND METHODS

### Definitions of the Analysis Population and Vaccine Preventable Outcomes (VPO)

Per Protocol Set (PPS) included all randomized participants who have consentedand have correctly received all the assigned vaccine doses (ETVAX or placebo) without major protocol deviations affecting efficacy endpoints up to the end of study surveillance period.

### Definition of Campylobacter Vaccine Preventable Outcome (VPO):

Moderate-to-severe acute diarrhea with stool sample containing *Campylobacter* regardless of ETEC infections or other co-infections.

Clinically-significant (moderate-to-severe) acute diarrhea is defined as a child brought to a clinic with four or more loose or liquid stools in the last 24 hours with moderate diarrhoea (e.g., vomiting ≤2 times/24 hours, drinks eagerly or thirsty, skin turgor ≤2 seconds, and restless/irritable) or severe diarrhea (e.g., hospitalized, intravenous (IV) fluids administered, unable to drink/drink poorly, vomiting≥3 times/24 hours, sunken eyes, lethargic/unconscious, or skin turgor ≥3 seconds).

Episodes of diarrhera were considered as vaccine preventable outcome if they further satisfied the criteria outlined below:
1. An episode that was moderate-to-severe diarrhea.
2. In at least one stool sample taken during the diarrhea episode (but before taking any of the prohibited medications), the following condition were met:
   - A rectal swab sample stored in a mNAT^{™} S Medium tube was positive for *Campylobacter jejuni* and/or *Campylobacter coli when* analyzed in the the Novodiag Bacterial GE test.

### Definition of EAEC Vaccine Preventable Outcome (VPO):

Moderate-to- severe diarrhea with stool sample containing EAEC regardless of ETEC infections or other co-infections.

Clinically-significant (moderate-to-severe) acute diarrhea is defined as a child brought to a clinic with four or more loose or liquid stools in the last 24 hours with moderate diarrhoea (e.g., vomiting ≤2 times/24 hours, drinks eagerly or thirsty, skin turgor ≤2 seconds, and restless/irritable) or severe diarrhea (e.g., hospitalized, intravenous (IV) fluids administered, unable to drink/drink poorly, vomiting≥3 times/24 hours, sunken eyes, lethargic/unconscious, or skin turgor ≥3 seconds).

Episodes of diarrhea were considered as vaccine preventable outcome if they further satisfied the criteria outlined below:
1. An episode that was moderate or severe diarhhea.
2. In at least one stool sample taken during the diarrhea episode (but before taking any of the prohibited medications), the following condition were met:
   A rectal swab sample stored in a mNAT^{™} S Medium tube was positive for EAEC when analyzed in the the Novodiag Bacterial GE test.

### Diagnostic measurements

### Bacterial Screening

When a rectal swab had been taken, it was placed immediately to an mNat^{™} S Medium tube. The tube was closed, mixed by shaking gently, and left on table at room temperature for 30 minutes. After this 500 µl sample was taken from the mNAT^{™} S Medium tube to Novodiag Bacterial GE test.

### Statistical methods & definition

Vaccine protective efficacy was derived from incidence ratios of diarrhea episodes with confidence intervals derived from 95% exact unconditional confidence limits of risk ratio, and p-values from Fisher's exact test.

## Claims

1. An oral vaccine comprising effective amounts of:
a. *E. coli* colonization factor antigens comprising CFA/I, CS3, CS5 and CS6 antigens, present on inactivated whole *Escherichia coli* cells;
b. a heat labile enterotoxin B subunit antigen being an *LCTBA* protein; and
c. an adjuvant being double mutant (R192G/ L211A) of *E. coli* heat-labile toxin (dmLT);
for use in inducing protective immunity against diarrheal disease caused by *Campylobacter spp.* and/or enteroaggregative *Escherichia coli* (EAEC).

2. The vaccine for use according to claim 1, wherein the disease is caused by *Campylobacter spp.*

3. The vaccine for use according to claim 2, wherein the disease is caused by *Campylobacter jejuni.*

4. The vaccine for use according to claim 2, wherein the disease is caused by *Campylobacter coli.*

5. The vaccine for use according to claim 1, wherein the disease is caused by EAEC.

6. The vaccine for use according to any of the preceding claims, wherein the vaccine comprises:
a. dmLT in an amount of at least 1.0 µg per unit dose;
b. *LCTBA* protein in an amount of at least 0.1 mg per unit dose;
c. CFA/I in an amount of at least 0.04 mg per unit dose;
d. CS3 in an amount of at least 0.13 mg per unit dose;
e. CS5 in an amount of at least 0.03 mg per unit dose; and
f. CS6 in an amount of at least 0.006 mg per unit dose.

7. The vaccine for use according to any of the preceding claims, wherein the vaccine comprises inactivated whole *Escherichia coli* cells in an amount of 1.0 x 10¹⁰ to 12 x 10¹⁰ cells per unit dose.

8. The vaccine for use according to any of the preceding claims, wherein the vaccine comprises, per unit dose:
a. 4.0 x 10¹⁰ to 12 x 10¹⁰ inactivated whole *Escherichia coli* cells comprising the following antigens:
i. 0.3-1.6 mg CFA/I;
ii. 1.0-5.2 mg CS3;
iii. 0.2-1.1 mg CS5; and
iv. 0.05-0.24 mg CS6;
b. 0.8-1.2 mg of LCBTA protein; and
c. 8-14 µg of dmLT adjuvant.

9. The vaccine for use according to claim 8, for use in immunizing human subjects of at least 6 years of age.

10. The vaccine for use according to any of claims 1-7, wherein the vaccine comprises, per unit dose:
a. 2.0x 10¹⁰ to 6.0 x 10¹⁰ inactivated whole *Escherichia coli* cells comprising the following antigens:
i. 0.15-0.8 mg CFA/I;
ii. 0.5-2.6 mg CS3;
iii. 0.1-0.55 mg CS5; and
iv. 0.025-0.12 mg CS6;
b. 0.4-0.6 mg of LCBTA protein; and
c. 4-7 µg of dmLT adjuvant.

11. The vaccine for use according to claim 10, for use in immunizing human subjects of 2-5 years of age.

12. The vaccine for use according to any of claims 1-7, wherein the vaccine comprises, per unit dose:
a. 1.0x 10¹⁰ to 3.0 x 10¹⁰ inactivated whole *Escherichia coli* cells comprising the following antigens:
i. 0.075-0.4 mg CFA/I;
ii. 0.25-1.3 mg CS3;
iii. 0.05-0.275 mg CS5; and
iv. 0.0125-0.06 mg CS6;
b. 0.2-0.3 mg of LCBTA protein; and
c. 2-3.5 µg of dmLT adjuvant.

13. The vaccine for use according to claim 12, for use in immunizing human subjects of 6-23 months of age.

14. The vaccine for use according to any of the preceding claims, wherein the vaccine is for administration in at least two doses separated in time by 7-30 days, preferably 14±7 days to a subject.

15. The vaccine for use according to any of claims 1-13, wherein the vaccine is for administration in at least three doses to a subject, wherein the day of the first dose is denoted day 1, the second dose is administered on day 8-42 following the first dose and the third dose is administered on day 50-130 following the first dose, with the proviso that the third dose is administered at least 30 days after the second dose.
